# EUROPÄISCHE PATENTSCHRIFT

(11) **EP 1 827 246 B1**
(45) Veröffentlichungstag und Bekanntmachung des Hinweises auf die Patenterteilung: **23.02.2011**
(21) Anmeldenummer: 05818492.0
(22) Anmeldetag: 15.11.2005
(51) Int. Cl.: A61B 17/04

(54) **ENDOSKOPISCHE NÄHMASCHINE**
ENDOSCOPIC STITCHING MACHINE
MACHINE A SUTURE ENDOSCOPIQUE

(30) Priorität: 18.11.2004 DE 102004056204
(43) Veröffentlichungstag der Anmeldung: 05.09.2007
(73) Patentinhaber: Karl Storz GmbH & Co. KG, 78532 Tuttlingen (DE)
(72) Erfinder: KLUNDT, Kurt, 67732 Hirschhorn (DE); MOLL, Philipp, 52076 Aachen (DE)
(74) Vertreter: Klein, Friedrich Jürgen
(86) Internationale Anmeldenummer: PCT/EP2005/012224
(87) Internationale Veröffentlichungsnummer: WO 2006/053710

(56) Entgegenhaltungen:
- DE-A1- 10 116 171
- GB-A- 872 952
- US-A- 5 593 402
- US-A- 5 947 996
- US-A- 6 126 359

## Beschreibung

Die Erfindung betrifft eine endoskopische Nähmaschine nach dem Oberbegriff des Patentanspruches 1.

In der DE 101 16 171 A1 ist eine chirurgische Nähmaschine offenbart, die insbesondere zur Herstellung von Nähten innerhalb des Körpers von Menschen oder Tieren dient. Da Nähmaschinen dieser Art zumindest teilweise in den menschlichen oder tierischen Körper eingeführt werden müssen, wird ein möglichst geringes Volumen für die in den Körper einzuführenden Bauteile angestrebt. Aus diesem Grund sind nicht nur die eigentlichen Bedienelemente, sondern möglichst auch die Antriebe für die Stichbildewerkzeuge wie Nadel, Greifer und Niederhalter in einem außerhalb des Körpers verbleibenden Gehäuse angeordnet. Die von den verschiedenen Antrieben erzeugten Bewegungen werden daher mittels geeigneten und von einem Gehäuseschaft aufgenommenen Übertragungsmitteln auf die am Ende des Gehäuseschaftes angeordneten Stichbildewerkzeuge übertragen.

Die sich hierdurch ergebende große Baulänge des Gehäuseschaftes und auch das die verschiedenen Antriebe aufnehmende Gehäuse erschweren daher in großem Maß das nach jedem Eingriff notwendige Sterilisieren der mit dem Körper in Berührung kommenden Teile der chirurgischen Nähmaschine.

Der Erfindung liegt daher die Aufgabe zu Grunde, für eine gattungsgemäße Nähmaschine unter Beibehaltung des Funktionsabstandes zwischen dem Gehäuse und den Stichbildewerkzeugen eine deren Sterilisieren erleichternde Lösung zu schaffen.

Diese Aufgabe wird ausgehend von einer gattungsgemäßen Nähmaschine dadurch gelöst, daß der Gehäuseschaft und die Übertragungsmittel innerhalb jeweils quer zu ihrer Längsrichtung verlaufenden Ebenen geteilt sind, und die jeweiligen (unteren und oberen) Abschnitte der Übertragungsmittel mittels je eines Kupplungsstückes lösbar miteinander verbindbar sind, wobei die Kupplungsstücke innerhalb entsprechender Ausnehmungen der Übertragungsmittel parallel zur Schnittebene verschiebbar und in zur Längsrichtung der Übertragungsmittel verlaufender Richtung formschlüssig in diesen aufgenommen sind.

Unter dem Begriff "Übertragungsmittel" sind hierbei diejenigen Teile der chirurgischen Nähmaschine zu verstehen, die geeignet und bestimmt sind, die vom jeweiligen Antrieb mittel- oder unmittelbar erzeugten Bewegungen für die einzelnen Stichbildewerkzeuge (wie beispielsweise Nadel, Greifer Niederhalter) auf diese zu übertragen. Dies können bei relativ kurzem Funktionsabstand zwischen den Stichbildewerkzeugen und dem Gehäuse die Nadelstange, Greiferwelle und die Druckstange für den Niederhalter selbst, oder auch zusätzliche Übertragungsmittel sein, die zwischen der Nadelstange, der Greiferwelle und der Druckstange für den Niederhalter einerseits und den entsprechenden Abgängen der einzelnen Antriebe andererseits vorgesehen sind.

Auf diese Weise wird zwischen den jeweiligen unteren und oberen Abschnitten der Übertragungsmittel bzw der Nadelstange, der Greiferwelle und der Druckstange für den Niederhalter eine Art Steckverbindung geschaffen, die unter Gewährleistung einer stets gleichen Gesamtlänge der Übertragungsmittel und der Kupplungsstücke ein werkzeugloses Lösen und Wiederherstellen der Verbindung der beiden Abschnitte des jeweiligen Übertragungsmittels bzw der Nadelstange, der Greiferwelle und der Druckstange für den Niederhalter ermöglicht.

Sofern hierbei das Lösen und Fügen des die Antriebsbewegung für die Nadel zu dieser weiterleitenden Übertragungsmittels bei sich im oberen Totpunktbereich seiner Bewegungsbahn befindlichem Übertragungsmittel erfolgt, kann diese Position dieses Übertragungsmittels als Referenzposition für das Lösen und Fügen der übrigen Übertragungsmittel dienen, wodurch insbesondere beim Fügen der Übertragungsmittel die Relativlage zwischen den mit dem jeweiligen Antrieb verbundenen Abschnitten der Übertragungsmittel und den die Stichbildewerkzeuge tragenden Abschnitten der Übertragungsmittel vorgegeben und damit stets ein positionsgerechtes Fügen der jeweiligen Abschnitte der Übertragungsmittel ohne Zuhilfenahme von Hilfsmitteln gewährleistet ist.

Eine in konstruktiver Hinsicht günstige Ausführung der Kupplungsstücke, die zur Übertragung sowohl von Längs- als auch Drehbewegungen der Übertragungsmittel geeignet sind, wird dann erreicht, wenn diese ein Mittelteil aufweisen, an dessen stirnseitigen Enden jeweils ein quer zur Längsrichtung der Übertragungsmittel gerichtetes Formteil vorgesehen ist, das in eine seiner Querschnittsform entsprechende Ausnehmung des Übertragungsmittels einführbar ist.

Um beim Fügen der Übertragungsmittel einen Mittenversatz auszuschließen und/oder einen durch Fertigungsungenauigkeiten vorhandenen Mittenersatz ausgleichen zu können, schließen die Längsachsen der beiden Formteile eines jeden Kupplungsstückes einen Winkel von 90 Grad ein.

Dabei ist es ferner vorteilhaft, wenn die Formteile jeweils einen prismatischen und einen an diesen anschließenden, im wesentlichen zylindrischen Bereich aufweisen und die Ausnehmungen der Übertragungsmittel komplementär hierzu gestaltet sind.

Zur Längs- und Quersicherung der beiden Hälften des Gehäuseschaftes ist dessen Trennung zur Bildung von gegenseitigen Anlageflächen stufenförmig ausgebildet, wobei im Trennbereich des einen Schaftteiles Positioniermittel für den anderen Schaftteil vorgesehen sind, die in am Trennbereich des anderen Schaftteiles vorgesehene Öffnungen ragen. Die Positioniermittel können hierbei von Paßstiften gebildet sein, die in entsprechende Bohrungen des anderen Schaftteiles eingreifen. Hierdurch wird einerseits das Fügen der beiden Schaftteile erleichtert und andererseits bilden die Positioniermittel zusammen mit den Bohrungen gleichzeitig eine Längssicherung der beiden Schaftteile.

Um sowohl die Lage der beiden Schaftteile als auch die Relativlage der Kupplungsstücke zu den Abschnitten der Übertragungsmittel in radialer Richtung zu sichern, ist der Gehäuseschaft von einem zu ihm konzentrisch angeordneten Zentrierrohr umgeben, das im Bereich seines gehäuseseitigen Endes mittels einer Rastvorrichtung lösbar mit dem oberen Schaftteil verbindbar ist.

Dabei ist es vorteilhaft, wenn die Rastvorrichtung einen im Gehäuseschaft radial bewegbaren federbelasteten Rastbolzen aufweist, der in eine am gehäuseseitigen Ende des Zentrierrohres ausgebildete Ausnehmung einrastet.

Um beim Fügen der Abschnitte der Übertragungsmittel das Auffinden der richtigen Relativlage derselben zu erleichtern und auch zu sichern, sind im Umfangsbereich des unteren Schaftteiles zwei quer zu diesem und parallel zueinander gerichtete Bohrungen zur Aufnahme einer Positionierhilfe vorgesehen, die gleichzeitig in am Umfang der unteren Abschnitte der Übertragungsmittel eingreifen.

Weitere Vorteile und Einzelheiten der Erfindung ergeben sich an Hand der nachfolgenden Beschreibung eines in der beigefügten Zeichnung dargestellten Ausführungsbeispieles derselben.

Es zeigt:
- **Fig. 1:**: eine schematische Gesamtansicht einer endoskopischen Nähmaschine;
- **Fig. 1a:**: eine vergrößerte Darstellung des die Stichbildewerkzeuge aufnehmenden unteren Bereiches des Schaftes;
- **Fig.**: **1b:**eine vergrößerte Darstellung des oberen Schaftbereiches mit dem Zentrierrohr;
- **Fig. 2:**: eine schaubildliche Darstellung des oberen Schaftteiles zu- sammen mit den oberen und unteren Übertragungsmitteln;
- **Fig. 3:**: eine schaubildliche Darstellung des unteren Schaftteiles mit den unteren Überragungsmitteln und der Positionierhilfe;
- **Fig. 4:**: eine Einzeldarstellung eines Übertragungsmittels mit einem Kupplungsstück;
- **Fig.**: **4a**: eine vergrößerte Darstellung der Einzelheit "A" der Fig.4;

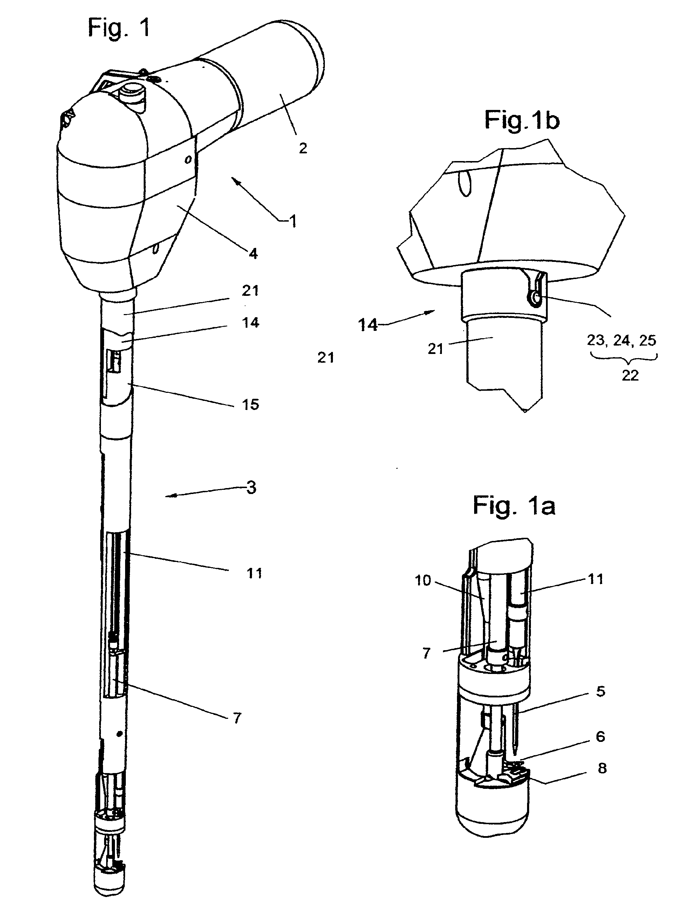

Die in Fig. 1 schematisch dargestellte endoskopische Nähmaschine entspricht in ihrem grundsätzlichen Aufbau der in der DE 101 16 171 A1 beschriebenen Nähmaschine.

Dementsprechend weist die erfindungsgemäße Nähmaschine ein im wesentlichen L-förmiges Gehäuse 1 auf, das von einem als Hohlkörper ausgebildeten Griffteil 2, einem zu diesem im wesentlichen senkrecht gerichteten Gehäuseschaft 3 und einem diesen mit dem Griffteil 2 verbindenden Gehäusemittelteil 4 gebildet ist.

Innerhalb des Griffteiles 2 ist ein nicht dargestellter Elektromotor angeordnet, der vorzugsweise als Gleichstrommotor ausgebildet ist. Von der nicht dargestellten Abtriebswelle des Gleichstrommotors wird über eine Kupplung und ein Untersetzungsgetriebe eine Hauptwelle angetrieben, von der die Bewegungen für eine Nadel 5 und einen Greifer 6 sowie für einen Niederhalter 8 abgeleitet werden. Die Ausbildung der hierzu dienenden Getriebe ist in der vorgenannten DE 101 16 171 A1 im Detail beschrieben, sodaß deren Beschreibung -so weit sie für das Verständnis der vorliegenden Erfindung von Interesse ist- als Bestandteil der Beschreibung der vorliegenden Erfindung anzusehen ist.

Demgmäß weist das Getriebe einen Nadelantrieb auf, der über ein Übertragungsmittel 9 mit der die Nadel 5 tragenden Nadelstange 11 in Antriebsverbindung steht und der Nadel 5 eine auf- und abgehende Bewegung erteilt.

Ferner weist das Getriebe einen Schwingantrieb für den sowohl eine Hubbewegung als auch eine Schwingbewegung ausführenden Greifer 6 auf. Der Schwingantrieb ist über ein Übertragungsmittel 12 mit der Greiferwelle 10 kinematisch verbunden und erteilt dieser, sowohl eine Hubbewegung als auch eine Schwingbewegung. Das Übertragungsmittel 12 führt somit eine aus einer Hub- und einer Schwingkomponente zusammengesetzte Hub-/ Schwingbewegung aus und überträgt somit sowohl ein wechselndes Drehmoment, als auch Zug- und Druckkräfte.

Schließlich wird vom Nadelantrieb während der ausgestochenen Phase der Nadel 5 eine Hubbewegung des Niederhalters 8 abgeleitet, um hierdurch zumindest während eines Teils der ausgestochenen Phase der Nadel 5 den Niederhalter 8 zur Erleichterung der Vorschubbewegung des Nähgutes um einen bestimmten Betrag periodisch anheben zu können. Hierzu steht mit dem entsprechenden (nicht dargestellten) Abtriebsglied des Nadelantriebes ein Übertragungsmittel 13 in Antriebsverbindung, dessen Bewegungen auf eine mit dem Niederhalter 8 starr verbundene Druckstange 7 übertragen werden.

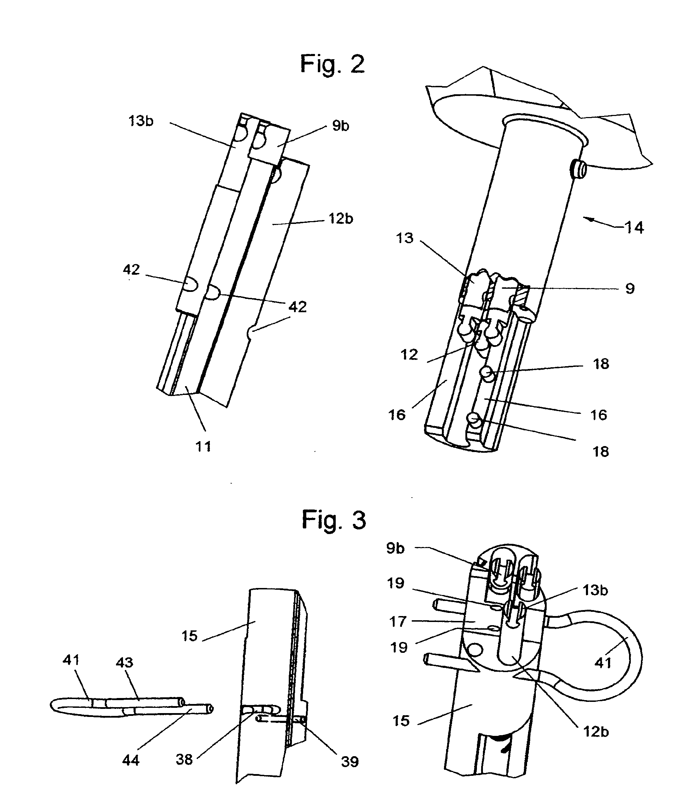

Wie aus den Fig. 2 und 3 hervorgeht, ist der rohrförmige Gehäuseschaft 3 durch eine im wesentlichen quer zu seiner Längsrichtung verlaufende Trennebene in einen oberen Schaftteil 14 und einen unteren Schaftteil 15 getrennt. Die Trennebene, die vorteilhafter Weise möglichst nahe am gehäuseseitigen Ende des Gehäuseschaftes vorgesehen ist, verläuft hierbei stufenförmig, wodurch an beiden Schaftteilen 14, 15 Anlageflächen 16, 17 gebildet sind. Hierdurch ist beim Fügen der beiden Schaftteile 14, 15 sowohl eine radiale als auch eine achsiale Ausrichtung vorgegeben. Im Bereich der Anlageflächen 16 des oberen Schaftteiles 14 sind an diesem von Paßstiften 18 gebildete Positioniermittel befestigt, die bei gefügtem Gehäuseschaft 3 in Bohrungen 19 ragen, die im Bereich der Anlageflächen 17 in den unteren Schaftteil 15 eingearbeitet sind. Damit ist eine Lagesicherung der beiden Schaftteile 14, 15 in deren Längs- und Querrichtung gegeben. Die beiden Schaftteile 14, 15 sind in ihrem gefügten Zustand von einem Zentrierrohr 21 umgeben, dessen oberes Ende mittels einer Rastvorrichtung 22 mit dem oberen Schaftteil 14 des Gehäuseschaftes 3 lösbar verbindbar ist. Die Rastvorrichtung 22 weist einen im oberen Schaftteil 14 radial verschiebbar angeordneten und unter der Wirkung einer (nicht dargestellten) Feder nach außen gedrückten Rastbolzen 23 auf, der bei auf den Gehäuseschaft 3 aufgeschobenem Zentrierrohr 21 in eine an diesem vorgesehene Ausnehmung 24 einrastet. Diese ist von einer im wesentlichen dem Durchmesser des Rastbolzens 23 entsprechenden Querboh-rung 25 gebildet, die zum Ende des Zentrierrohres 21 hin aufgeschlitzt ist.

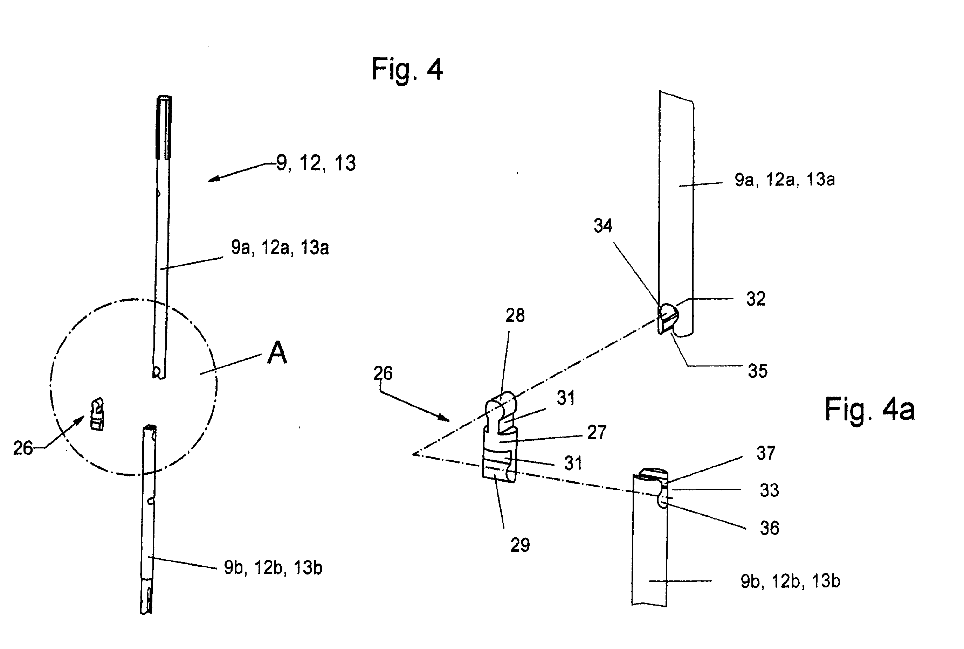

Die mit ihrem zugehörigen Antrieb verbundenen und in den Gehäuseschaft 3 geführten Übertragungsmittel 9, 12 und 13 sind in ihrem der Trennstelle des Gehäuseschaftes entsprechenden Bereich innerhalb einer quer zu ihrer Längsrichtung verlaufenden Ebene in jeweils zwei Abschnitte, nämlich in die jeweils oberen Abschnitte 9a, 12a und 13a und die jeweils unteren Abschnitte 9b, 12b und 13b unterteilt und sind mittels jeweils einem Kupplungsstück 26 lösbar miteinander verbindbar (Fig. 4,4a). Sämtliche Kupplungsstücke 26 weisen eine einheitliche Bauform auf und sind daher mit dem Bezugszeichen 26 versehen. Jedes der Kupplungsstücke 26 weist ein Mittelteil 27 auf, dessen Querschnittsform dem Querschnitt des jeweiligen Übertragungsmittels 9, 12 und 13 entspricht. Bei der in der Zeichnung dargestellten beispielhaften Ausführungsform der Erfindung weisen alle Übertragungsmittel 9, 12 und 13 einheitlich einen kreisförmigen Querschnitt auf, sodaß auch sämtliche Mittelteile 27 der Kupplungsstücke 26 zylinderförmig ausgebildet sind, wobei deren Durchmesser etwas geringer als derjenige der Übertragungsmittel ist. Im Bereich seiner beiden stirnseitigen Enden ist jedes Kupplungsstück 26 mit insgesamt zwei Formteilen 28, 29 versehen, die beim gezeigten Ausführungsbeispiel im wesentlichen zylinderförmig gestaltet und über jeweils einen Steg 31 mit dem Mittelteil 27 verbunden sind. Wie hierzu insbesondere aus Fig. 4a der Zeichnung hervorgeht, schließen die beiden Formteile 28, 29 eines jeden Kupplungsstückes 26 einen Winkel von vorzugsweise 90 Grad ein, sodaß hierdurch ein Mittenversatz der beiden Abschnitte der Übertragungsmittel ausgeglichen werden kann.

Die mit Hilfe der Kupplungsstücke 26 miteinander zu verbindenden Abschnitte der Übertragungsmittel weisen an ihren miteinander zu verbindenden Enden Ausnehmungen 32, 33 auf, deren Form der Form der Formteile 28, 29 sowie der Stege 31 entspricht. Dementsprechend ist in den jeweils unteren Bereichen der jeweils oberen Abschnitte 9a, 12a und 13a der Übertragungsmittel 9, 12 und 13 jeweils eine Querbohrung 34 vorgesehen, die in einen zur Stirnseite des jeweiligen Übertragungsmittels hin offenen Schlitz 35 mündet, dessen Breite der Breite des Steges 31 angepaßt ist. Die Form der Ausnehmungen 32 ist daher durch die Querbohrung 34 und den Schlitz 35 bestimmt.

Analog hierzu ist in den jeweils oberen Bereichen der jeweils unteren Abschnitte 9b, 12b und 13b der Übertragungsmittel 9, 12 und 13 jeweils eine, zur Querbohrung 34 im Winkel von 90 Grad verlaufende Querbohrung 36 vorgesehen, die in einen zur Stirnseite des jeweiligen Übertragungsmittels hin offenen Schlitz 37 mündet, dessen Breite ebenfalls der Breite des Steges 31 angepaßt ist. Die Form der Ausnehmungen 33 ist daher ebenfalls durch die entsprechende Querbohrung 36 und den Schlitz 37 bestimmt.

Damit sind die Kupplungsstücke 26 in den Ausnehmungen 32, 33 zum Ausgleich von Fertigungsungenauigkeiten oder eines Mittenversatzes in radialer Richtung verschiebbar, in axialer Richtung jedoch aufgrund der Formgebung der Ausnehmungen 32, 33 sowie der Formteile 28, 29 formschlüssig gehalten. Da bei durch die Kupplungsstücke 26 miteinander verbundenen oberen und unteren Abschnitten der Übertragungsmittel und gefügtem unteren und oberen Schaftteil das Zentrierrohr 21 über den Gehäuseschaft 3 geführt und mittels der Rastvorrichtung 22 mit diesem verbunden ist, sind die Kupplungsstücke 26 auch in radialer Richtung formschlüssig gesichert. Dies bedeutet, daß der Gehäuseschaft 3 und die in seinem Inneren angeordneten Teile eine Baueinheit bilden, die ohne Schraubverbindungen zusammen gehalten wird und zum Zweck des Sterilisierens ohne Zuhilfenahme von Werkzeugen in ihre Bestandteile zerlegt und wieder gefügt werden kann.

In einer bevorzugten Ausführungsform der Erfindung können die beiden Formteile 28, 29 innerhalb der jeweiligen Ausnehmung 32, 33 mit leichtem Schiebesitz bewegbar sein. Dies ist insbesondere zum Ausgleich von Mittenversatz und/oder nicht vermeidbaren Fertigungstoleranzen von Bedeutung.

Da somit die den jeweils oberen Abschnitten 9a, 12a und 13a der Übertragungsmittel zugehörenden Formteile 28 der Kupplungsstücke 26 nach deren erstmaligem Fügen diese Winkellage selbst bei sich anschließendem mehrmaligen Trennen und jeweils erneutem Fügen der Übertragungsmittel stets wieder einnehmen, ist damit auch die Lage der den jeweils unteren Abschnitten 9b, 12b und 13b der Übertragungsmittel zugeordneten Formteile 29 der Kupplungsstücke 26 vorgegeben.

Dies bedeutet, daß beim Fügen der jeweils unteren Abschnitte 9b, 12b und 13b der Übertragungsmittel deren Relativlage zu den oberen Abschnitten 9a, 12a und 13a vorgegeben ist, d.h. die unteren Abschnitte 9b, 12b und 13b in eine Lage zu bringen sind, in der sie über die Formteile 29 geschoben werden können, wobei deren zylindrischer Bereich in die jeweilige Querbohrung 36 eintaucht und die Stege 31 in den jeweiligen Schlitz 37 gleiten.

Um das Auffinden und Sichern der Fügeposition der unteren Übertragungsmittel 9b, 12b und 13b zu erleichtern, sind im Umfangsbereich des unteren Schaftteiles 15 zwei quer zu dessen Längsachse und parallel zueinander gerichtete Bohrungen 38, 39 vorgesehen, die im unteren Schaftteil 15 einseitig offene Ausnehmungen mit vorzugsweise halbkreisförmigem Querschnitt bilden. Entsprechende Ausschnitte 42 sind an den unteren Übertragungsmitteln 9b, 12b und 13b vorgesehen, die in der Fügeposition der unteren Übertragungsmittel 9b, 12b und 13b mit den durch die Bohrungen 38,39 im unteren Schaftteil 15 gebildeten Ausnehmungen zylindrische Öffnungen bilden. Die Lage der Ausschnitte 42 an den unteren Übertragungsmitteln 9b, 12b und 13b einerseits und die Lage der Bohrungen 38,39 im unteren Schaftteil 15 andererseits ist dabei so gewählt, daß beim Fluchten der Ausschnitte 42 mit den Bohrungen 38, 39 die jeweiligen unteren Formteile 29 und die Stege 31 der Kupplungsstücke 26 parallel zur zugehörigen Bohrung 36 bzw zum entsprechenden Schlitz 37 gerichtet und in diese einführbar sind.

Die jeweiligen Ausschnitte 42 und die Bohrungen 38, 39 dienen zur Aufnahme zweier im Abstand und parallel zueinander angeordneter Schenkel 43, 44 einer Positionierhilfe 41 mit der die Fügeposition der unteren Übertragungsmittel 9b, 12b und 13b bis zum Überschieben des Zentrierrohres 21 über den Bereich der Trennstelle des Schaftes geführt ist.

## Patentansprüche

1. Endoskopische Nähmaschine mit einem Gehäuse zur Aufnahme von Antrieben für die Stichbildewerkzeuge wie Nadel, Greifer und Niederhalter, einem sich an dieses anschließenden Gehäuseschaft zur Aufnahme von Mitteln zur Übertragung der von den Antrieben erzeugten Bewegungen auf die Stichbildewerkzeuge, die mindestens eine auf- und abgehende fadenführende Nadel und einen mit dieser zusammenwirkenden, pro Stichbildezyklus mindestens eine Schwingbewegung ausführenden Greifer umfassen, **dadurch gekennzeichnet, daß**
der Gehäuseschaft (3) und die Übertragungsmittel (9,12,13) innerhalb einer jeweils quer zu ihrer Längsrichtung verlaufenden Ebene in einen unteren und einen oberen Schaftteil (14,15) und in untere und obere Übertragungsmittel (9a,12a,13a, bzw. 9b,12b,13b) geteilt sind, und die unteren und oberen Übertragungsmittel (9a,12a,13a, und 9b,12b, 13b) mittels je eines Kupplungsstückes (26) lösbar miteinander verbindbar sind, wobei die Kupplungsstücke (26) innerhalb entsprechender Ausnehmungen (32,33) der unteren und oberen Übertragungsmittel (9a,12a,13a, bzw. 9b,12b,13b) parallel zur Schnittebene verschiebbar und in zur Längsrichtung der Übertragungsmittel (9, 12, 13) verlaufender Richtung formschlüssig in diesen aufgenommen sind.

2. Endoskopische Nähmaschine nach Anspruch 1, **dadurch gekennzeichnet, daß** die Kupplungsstücke (26) jeweils zwei quer zur Längsrichtung der Übertragungsmittel (9,12,13) gerichtete Formteile (28,29) aufweisen und beide Formteile (28,29) in ihrer Querschnittsform entsprechende Ausnehmungen (32,33) der Übertragungsmittel (9, 12,13) einführbar sind.

3. Endoskopische Nähmaschine nach Anspruch 1 oder 2, **dadurch gekennzeichnet, daß** die Längsachsen der beiden Formteile (28,29) eines jeden Kupplungsstückes (26) einen Winkel von 90 Grad einschließen.

4. Endoskopische Nähmaschine nach Anspruch 2, **dadurch gekennzeichnet, daß** die Formteile (28,29) jeweils einen prismatischen und einen an diesen anschließenden, im wesentlichen zylindrischen Bereich aufweisen und die Ausnehmungen (32,33) der Übertragungsmittel (9,12,13) komplementär hierzu gestaltet sind.

5. Endoskopische Nähmaschine nach Anspruch 1 **dadurch gekennzeichnet, daß** die Trennung des Gehäuseschaftes (3) zur Bildung von gegenseitigen Anlageflächen (16,17) stufenförmig ausgebildet ist und im Trennbereich des einen Schaftteiles (14) Positioniermittel (18) für den anderen Schaftteil (15) vorgesehen sind, die in am Trennbereich des anderen Schaftteiles (15) vorgesehene Öffnungen (19) ragen.

6. Endoskopische Nähmaschine nach Anspruch 1 **dadurch gekennzeichnet, daß** der Gehäuseschaft (3) von einem zu ihm konzentrisch angeordneten Zentrierrohr (21) umschlossen ist.

7. Endoskopische Nähmaschine nach Anspruch 6 **dadurch gekennzeichnet, daß** das Zentrierrohr (21) im Bereich seines gehäuseseitigen Endes mittels einer Rastvorrichtung (22) lösbar mit dem Gehäuseschaft (39 verbindbar ist.

8. Endoskopische Nähmaschine nach Anspruch 7 **dadurch gekennzeichnet, daß** die Rastvorrichtung (22) einen im Gehäuseschaft (3) radial bewegbaren federbelasteten Rastbolzen (23) aufweist, der in eine am gehäuseseitigen Ende des Zentrierrohres (21) ausgebildete Ausnehmung (24) einrastet.

9. Endoskopische Nähmaschine nach Anspruch 1 **dadurch gekennzeichnet, daß** im Umfangsbereich des unteren Schaftteiles (15) zwei quer zu diesem und parallel zueinander gerichtete Bohrungen (38,39) zur Aufnahme einer Positionierhilfe (41) vorgesehen sind, die gleichzeitig in Ausschnitte (42) der Übertragungsmittel (9,12,13) eingreifen.

## Claims

1. An endoscopic stitching machine comprising a housing for mounting drives for the stitch-forming tools such as needles, grippers and pressure pads, an adjoining housing shaft for accommodating means for transmitting the movements generated by the drives to the stitch-forming tools, which at least comprise at least one upward- and downward-moving thread-carrying needle and at least one gripper which cooperates therewith and executes at least one swinging movement per stitch-forming cycle, **characterised in that** the housing shaft (3) and the transmission means (9, 12, 13) are divided within a plane each running transversely to their longitudinal direction into an upper and a lower shaft part (14, 15) and into lower and upper transmission means (9a, 12a, 13a or 9b, 12b, 13b) and the lower and upper transmission means (9a, 12a, 13a and 9b, 12b, 13b) are detachably connected to each other by means of respectively one coupling piece (26), wherein the coupling pieces (26) can be introduced into and positively housed within corresponding recesses (32, 33) of the lower and upper transmission means (9a, 12a, 13a or 9b, 12b, 13b) parallel to the plane of intersection and running in the longitudinal direction of the transmission means (9, 12, 13).

2. The endoscopic stitching machine according to claim 1, **characterised in that** the coupling pieces (26) each have two shaped parts (28, 29) directed transversely to the longitudinal direction of the transmission means (9, 12, 13) and both shaped parts (28, 29) can be inserted into recesses (32, 33) of the transmission means (9, 12, 13) corresponding to their cross-sectional shape.

3. The endoscopic stitching machine according to claim 1 or 2, **characterised in that** the longitudinal axes of the two shaped parts (28, 29) of each coupling piece (26) encloses an angle of 90 degrees.

4. The endoscopic stitching machine according to claim 2, **characterised in that** the shaped parts (28, 29) each have a prismatic and an adjoining substantially cylindrical region and the recesses (32, 33) of the transmission means (9, 12, 13) are configured as complementary hereto.

5. The endoscopic stitching machine according to claim 1, **characterised in that** the separation of the housing shaft (3) to form mutual bearing surfaces (16, 17) is step-shaped and in the separating region of one shaft part (14) positioning means (18) are provided for the other shaft part (15), which project into openings (19) provided on the separating region of the other shaft part (15).

6. The endoscopic stitching machine according to claim 1, **characterised in that** the housing shaft (3) is enclosed by a centring tube (21) which is arranged concentrically to said shaft.

7. The endoscopic stitching machine according to claim 6, **characterised in that** in the area of its end on the housing side, the centring tube (21) is detachably connected to the housing shaft (39) by means of a locking device (22).

8. The endoscopic stitching machine according to claim 7, **characterised in that** the locking device (22) has a spring-loaded locking bolt (23) which can be moved radially in the housing shaft (3) which engages in a recess (24) formed at the end of the centring tube (21) on the housing side.

9. The endoscopic stitching machine according to claim 1, **characterised in that** in the circumferential area of the lower shaft part (15), two holes (38, 39) directed transversely to said part and parallel to one another are provided to receive a positioning aid (41), which holes simultaneously engage in cut-outs (42) of the transmission means (9, 12, 13).

## Revendications

1. Machine à coudre endoscopique comprenant un corps dans lequel sont logés les systèmes d'entraînement des outils de formation du point tels que l'aiguille, le boudeur et le pied presseur, d'une tige à la suite du corps formant boîtier dans lequel sont logés les systèmes de transmission des mouvements générés par les systèmes d'entraînement sur les outils de formation du point englobant au minimum une aiguille supportant le fil à coudre et effectuant un mouvement ascendant et descendant ainsi qu'un boucleur concourant avec l'aiguille et effectuant au minimum un mouvement oscillant par cycle de formation du point, **caractérisée par** ce que
la tige (3) formant boîtier et les moyens de transmission (9, 12, 13) sont divisés chacun à l'intérieur de leur plan transversalement par rapport à leur sens longitudinal en une partie de tige supérieure (14, 15) et en des éléments de transmission inférieurs et supérieurs (9a, 12a, 13a et 9b, 12b et 13b) et que les éléments de transmission inférieurs et supérieurs (9a, 12a, 13a et 9b, 12b et 13b) peuvent être raccordés de façon amovible au moyen d'accouplements (26), ces derniers pouvant coulisser parallèlement au plan de coupe dans des creux (32, 33) dans les éléments de transmission inférieurs et supérieurs (9a, 12a, 13a et 9b, 12b et 13b) et y être logés de façon positive dans le sens longitudinal des moyens de transmission (9, 12, 13).

2. Machine à coudre endoscopique selon la revendication 1, **caractérisée par le fait que** les accouplements (26) possèdent chacun deux pièces calibrées (28, 29) orientée transversalement par rapport au sens longitudinal des éléments de transmission (9, 12, 13) et que les deux pièces calibrées (28, 29), grâce à la forme de leur section, puissent être engagées dans les creux (32, 33) de section correspondante des éléments de transmission (9, 12, 13).

3. Machine à coudre endoscopique selon la revendication 1 ou 2, **caractérisée par le fait que** les axes longitudinaux des deux pièces calibrées (28, 29) de chaque accouplement (26) forment un angle de 90 degrés.

4. Machine à coudre endoscopique selon la revendication 2, **caractérisée par le fait que** les pièces calibrées (28, 29) présentent chacune une partie prismatique et, suite à celle-ci, une partie pour l'essentiel cylindrique et que les creux (32, 33) dans les éléments de transmission (9, 12 et 13) sont conçus de manière complémentaire à celle-ci.

5. Machine à coudre endoscopique selon la revendication 1, **caractérisée par le fait que** le plan de séparation de la tige à boîtier (3) prévu pour former des surfaces d'appui (16, 17) réciproques, est conçu en gradins et que dans le plan de séparation de l'une des parties de tige (14) sont prévus des moyens de positionnement (18) pour l'autre partie de tige (15) qui pénètrent dans les orifices (19) prévus dans le plan de séparation de tige (15).

6. Machine à coudre endoscopique selon la revendication 1, **caractérisée par le fait que** la tige (3) formant boîtier est entourée d'un tube de centrage (21) concentrique par rapport à la tige.

7. Machine à coudre endoscopique selon la revendication 6, **caractérisée par le fait qu'**au niveau de son extrémité côté boîtier, le tube de centrage (21) peut être relié de manière amovible par un dispositif d'arrêt (22) à la tige à boîtier (39).

8. Machine à coudre endoscopique selon la revendication 7, **caractérisée par le fait que** le dispositif à crans (22) est muni d'une goupille d'arrêt (23) commandé par ressort et mobile dans le sens radial de la tige à boîtier, qui s'encliquette dans un creux (24) formé dans l'extrémité côté boîtier du tube de centrage (21).

9. Machine à coudre endoscopique selon la revendication 1, **caractérisée par le fait que** le pourtour de la partie de tige inférieure (15) comprend deux perçages (38, 39) disposés transversalement par rapport à celle-ci et parallèlement l'un par rapport à l'autre et prévus pour accueillir un moyen de positionnement (41) qui s'engage simultanément dans les entailles (42) réalisés dans les éléments de transmission (9, 12 et 13).
